# EUROPEAN PATENT SPECIFICATION

(11) **EP 3 658 215 B1**
(45) Date of publication and mention of the grant of the patent: **18.08.2021**
(21) Application number: 19703781.5
(22) Date of filing: 09.01.2019
(51) Int. Cl.: A61M 25/04, A61M 25/00, A61F 2/24

(54) **A DEVICE FOR INSERTING A GUIDE WIRE IN A BLOOD VESSEL**
EINE VORRICHTUNG ZUM EINSETZEN EINES FÜHRUNGSDRAHTES IN EIN BLUTGEFÄSS
DISPOSITIF POUR INSERER UN FIL DE GUIDAGE DANS UN VAISSEAU SANGUIN

(30) Priority: 10.01.2018 IT 201800000671
(43) Date of publication of application: 03.06.2020
(73) Proprietor: Vivheart S.r.l., 20121 Milano MI (IT)
(72) Inventor: Montorfano, Matteo, 20122 Milano MI (IT)
(74) Representative: Barbaro, Gaetano
(86) International application number: PCT/IB2019/050155
(87) International publication number: WO 2019/138334

(56) References cited:
- WO-A1-2016/024235
- US-A1- 2014 207 179
- US-A1- 2015 223 757
- US-A1- 2017 042 678
- US-B1- 6 517 551

## Description

### TECHNICAL FIELD

The present disclosure relates in general to catheters and more in particular to a device for inserting a guidewire into a blood vessel, wherein the device is self-centering and may be inserted into an artery for directing a guidewire through a heart valve.

### BACKGROUND OF THE INVENTION

Percutaneous valve replacement (Transcatheter Aortic Valve Replacement or more briefly TAVR) is a technique performed by inserting a catheter containing a guidewire, which is used as a guide for the implantation of a cardiac valve prosthesis. The catheter is threaded through the femoral artery and pushed up to the proximity of the defective heart valve or through the chest, for exiting the guidewire contained in the catheter near the cardiac valve so that it passes throughout it. Once the guidewire has passed throughout the heart valve, it is used to guide a valve prosthesis to the heart, where it must be positioned.

A difficulty related to this type of technique consists in the fact that the operator must be particularly skilled in order to quickly insert the guidewire through the faulty valve.

The imaging equipment currently used in operating rooms only allow a two-dimensional view and not a three-dimensional view of the guidewire approaching the defective heart valve. As a consequence, from the two-dimensional image the operator may have the wrong impression of correctly directing the guidewire to the center of the heart valve, which is also narrow and degenerated, crooked, when instead the apical end of the guidewire is going to abut against the walls of the aorta.

In any case, even if a second apparatus for images arranged so as to provide a different view were available, there would be the problem of positioning and properly orienting a catheter while pushing forward the guidewire. Moreover, the guidewire is made of yielding material so as not to damage the tissues with which it comes into contact, so it bends easily even when it should remain straight to pass through the defective valve.

This problem is even more pronounced in the so-called Valve-In-Valve technique (or more briefly VIV), in which a defective valve prosthesis is not completely removed, but is used as a frame in which a new percutaneous heart valve prosthesis is anchored. This new technique prevents the patient from the trauma of removing the old prosthetic valve, which remains attached to the heart tissue while the new valve prosthesis is attached to the old. Nevertheless this is critical because the guidewire, if pushed against the damaged portions of the old heart valve prosthesis, may cause the detachment of a part of it.

US 2014/0207179 A1, upon the disclosure of which the preamble of claim 1 is drafted, discloses a catheter for insertion into vasculature of a patient to a target area in the vasculature which includes a hollow inner shaft, a non-occluding self-expandable scaffold coupled to the distal end of the inner shaft and disposed at the distal end of the inner shaft, and a hollow outer shaft. However, such a configuration is limited as it does not allow the user to control the direction, position and stability of the device, once it's inserted into the vasculature, especially in operations of large calibre blood vessels as the aorta.

### SUMMARY

Studies performed by the applicant led to the design of a new device comprising multiple catheters with different characteristics, one inserted inside the other, to direct a guided thread through a blood vessel and/or a heart valve in a stable, carefully controlled and repeatable manner.

The device of this disclosure solves the problem thanks to an outermost catheter, to an intermediate catheter inserted into the outermost catheter and to a more internal catheter inserted into the intermediate catheter, each with its own peculiar characteristics, as defined in claim 1. The intermediate catheter defines, at an apical portion, a tube on which it slides an anchoring element for anchoring to the inner walls of a blood vessel. The anchoring element comprises an annular slider sliding around the tube and a plurality of wire elements each having a proximal end attached to the tube and a distal end attached to the annular slider. The wire elements are made of a shape memory material and are configured to spontaneously bend away radially with respect to the tube to abut against the inner walls of the blood vessel at opposite points when they exit the outermost catheter, dragging the annular slider which is slid on the tube towards the proximal ends of the wire elements. The apical portion the innermost catheter is configured to assume a curved position at rest, and is configured to be elastically extended when inserted into the tube of the intermediate catheter and to spontaneously return to the curved position at rest as it exits from the tube.

Preferred or alternative embodiments are defined in the dependent claims.

### BRIEF DESCRIPTION OF THE DRAWINGS

Figure 1A is a front perspective view of a device of this disclosure for inserting a guidewire for heart valve prosthesis, in which the intermediate catheter and the inner catheter are in a retracted configuration with respect to the outer catheter.
Figure 1B is a rear perspective view of the device of figure 1A, in which the rear hole is shown in the handle of the innermost catheter, through which the guidewire is inserted.
Figure 2A shows the device of Figure 1A, with the innermost catheter in a retracted configuration and the intermediate catheter in advanced configuration with respect to the outermost catheter, for expanding an anchoring element which abuts against the inner walls of a blood vessel.
Figure 2B is a detailed view of the anchoring element of figure 2A, showing the slider of the anchoring element retracted around the tube of the intermediate catheter to bend the wire elements.
Figure 3A shows the device of Figure 2A, with the innermost catheter in an advanced configuration with respect to the outermost catheter to exit outside the intermediate catheter tube.
Figure 3B is a detailed view of the anchoring element of Figure 3A, showing the innermost catheter which spontaneously assumes a curved and orientable configuration when exiting from the intermediate catheter tube.
Figure 4A shows the device of Figure 3A, with the guidewire inserted through the innermost catheter.
Figure 4B is a detailed view of the anchoring element of Figure 4A, showing the guidewire oriented in the direction established by the innermost catheter.

### DETAILED DESCRIPTION

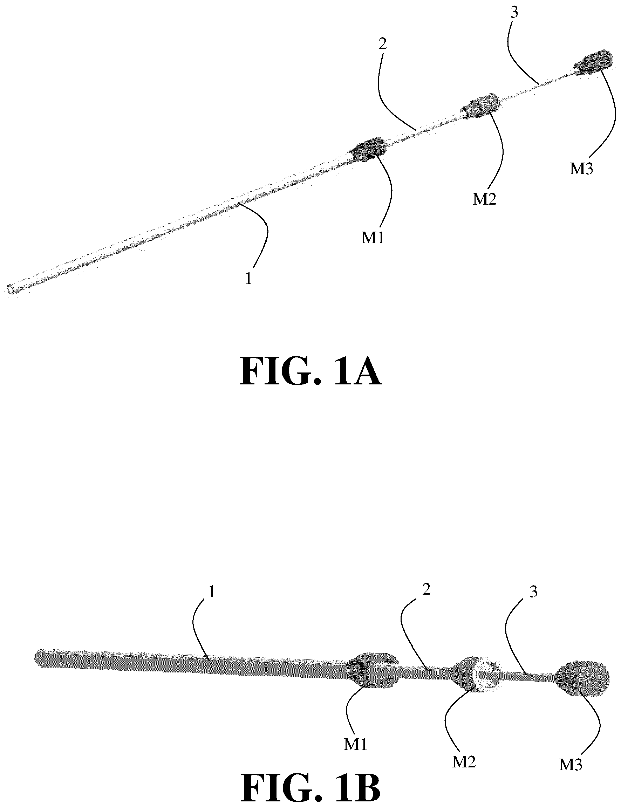

A device of this disclosure of a guidewire insertion through cardiac valve prosthesis is shown in Figures 1A and 1B. It is basically composed of three catheters inserted one inside the other: an outer catheter 1, an intermediate catheter 2 and an inner catheter 3. Conveniently, each of these three catheters may be rotated around its longitudinal axis and moved forward or backward by means of respective handles M1, M2 and M3. The three catheters 1, 2, 3, have different properties from each other and, thanks to the synergistic combination of these properties, allow the device of this disclosure to place a guidewire in a precise and easily reproducible way through a defective heart valve.

All catheters are made of suitable material and have adequate length to be inserted into an artery of the human body. Typically, they are intended to be threaded into the femoral artery and to be pushed up to the aorta until they attain a defective heart valve. Alternatively, they may be placed at the chest of a patient in a position as close as possible to the defective heart valve.

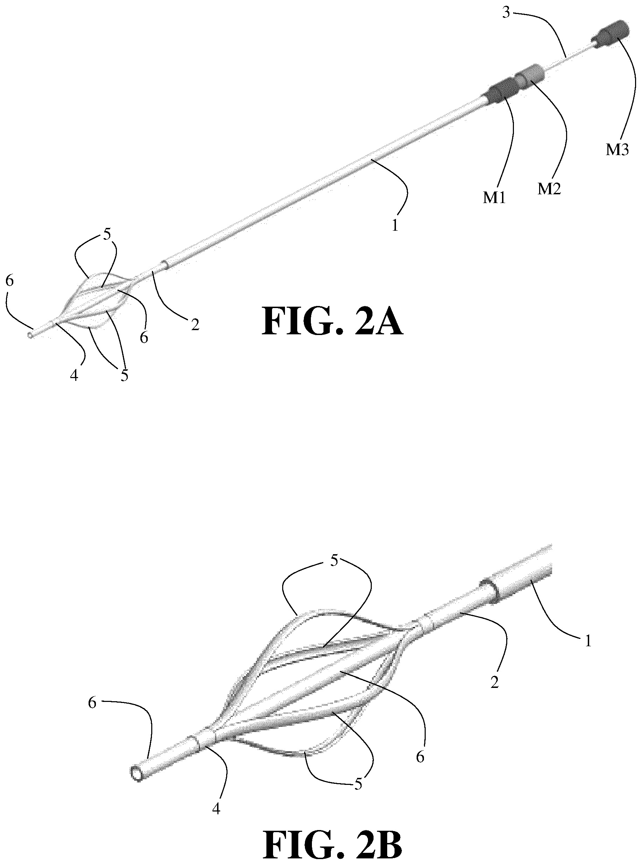

The outermost catheter 1 is a common catheter that may be inserted into an artery and its function is to convey the intermediate catheter 2 and the inner catheter 3, leaving them out only near the defective heart valve. When the apical end of the catheter 1 is near the defective heart valve, the handle M1 of the outer catheter 1 is retracted towards the handle M2 of the intermediate catheter 2, as shown in Figure 2A.

The intermediate catheter 2 has, at its apical part, an anchoring element to the walls of a blood vessel. This anchoring element substantially comprises an annular slider 4, which connects wire elements 5 of a shape memory self-expandable frame and that slides freely on the tube 6 of the catheter 2.

The detailed view of Figure 2B allows a better understanding of the structure of the self-expanding frame. As long as the intermediate catheter 2 is retracted into the outer catheter 1, the annular slider 4 is arranged around the tube 6 of the intermediate catheter 2 so that the wire elements 5 of the self-expanding frame are laying along the tube 6. When the handle M1 is retracted against the handle M2, the anchoring element protrudes from the outer catheter 1. The wire elements 5 are made of a shape-memory material, for example Nitinol, and tend to spontaneously bend as shown in Figure 2B when free, allowing the ring slider 4 to slide along the tube 6 as in a mechanism for opening an umbrella. The length of the wire elements 5 of the self-expanding frame will be appropriately determined so that they bend and abut against the walls of the blood vessel in which the frame has expanded. In this way, the catheter 2 may be anchored where desired within the vessel, providing a reference point.

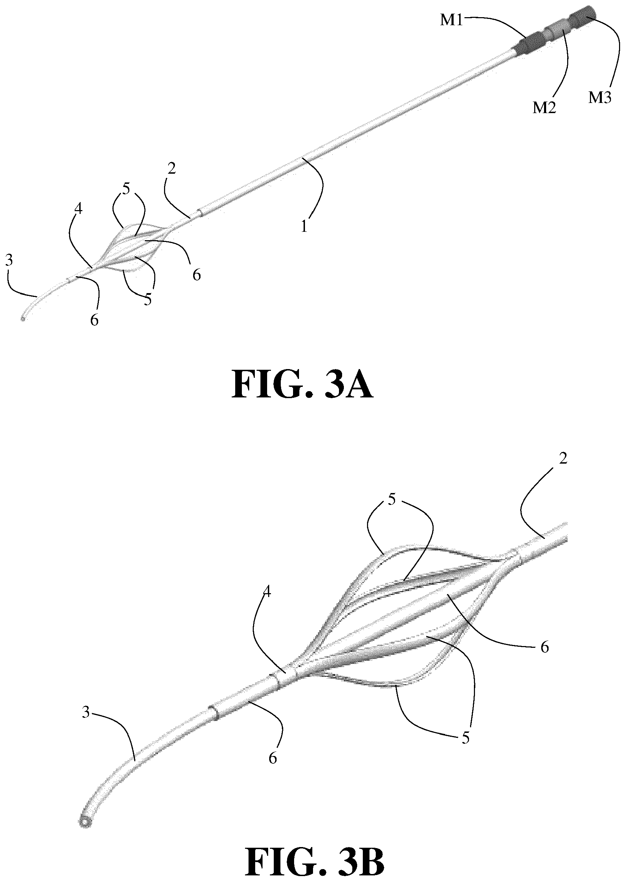

When the frame is against the blood vessel, the handle M3 is pushed towards the handle M2, as shown for example in Figure 3A and in the detail view of Figure 3B. The inner catheter 3 has at least one elastic apical part which, at rest, assumes a curved configuration.

When the apical part of the inner catheter 3 is inside the intermediate catheter 2, it is elastically forced to remain lying therein. As the handle M3 is pushed towards the handle M2, the apical part of the catheter 3 comes out of the tube 6 and the longer it protrudes from the tube 6 the more it bends. By pushing or retracting the handle M3 and by turning it on itself, the operator may adjust at will the curvature of the apical part of the catheter 3 and orient it in the blood vessel, in order to point it towards the center of the defective heart valve.

The intermediate catheter 2 is anchored to the walls of the blood vessel, thus the operator may easily maintain the orientation of the apical part of the catheter 3 by holding the M3 handle stationary for as long as desired. As a consequence, it is possible to use a unique imaging apparatus to verify the orientation of the apical portion of the catheter 3 according to a first point of view, then to move the same imaging apparatus to a different point of view to verify that the innermost catheter 3 is actually pointing at the center of the defective heart valve.

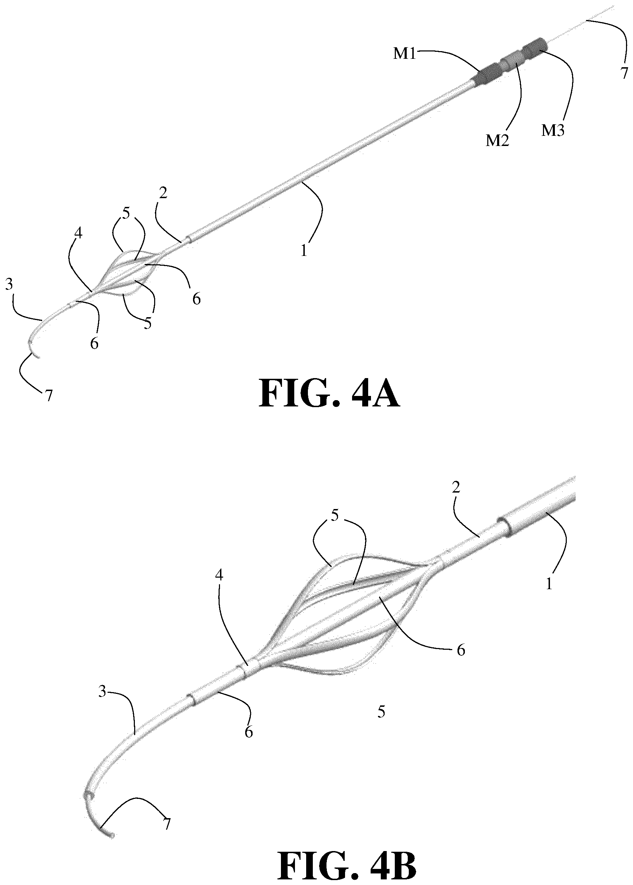

When the operator considers that the opening of the innermost catheter 3 is pointing precisely in the desired direction, it slides the guidewire 7 into the catheter 3 as shown in Figure 4A and in the detail view of Figure 4B. The guidewire 7, sliding in the catheter 3, may only be arranged in the desired direction since the first attempt, without any risk of damaging the walls of the blood vessel or of touching some delicate part of the defective heart valve.

Even the inner catheter 3, as well as the wire elements, may conveniently be made of a shape memory material, such as Nitinol.

Preferably, but not necessarily, the outermost catheter 1 will have a diameter not greater than 10 F (3.33mm), in order to be inserted inside a femoral artery, and the lumen of the inner catheter 3 must be such as to contain a guidewire of at least 0.035 inches (0.89mm). The lumen and the thickness of the walls of the intermediate catheter 2, as well as the thicknesses of the walls of the outermost catheter 1 and of the innermost catheter 3 will be easily determined accordingly by the expert technician to perform the functions illustrated above.

## Claims

1. A device for inserting a guidewire suitable for hearth valve prosthesis in a blood vessel, comprising:
an outer catheter (1),
an intermediate catheter (2) that may be inserted into said outer catheter (1),
an inner catheter (3) that may be inserted in said intermediate catheter (2),
wherein said intermediate catheter (2) has an apical portion that defines a tube (6) onto which an anchoring element for anchoring to inner walls of a blood vessel slides, said anchoring element comprises an annular slider (4) slidable around said tube (6) and a plurality of wire elements (5) each having a proximal end fixed to said tube (6) and a distal end fixed to said annular slider (4), wherein said wire elements (5) are made of a shape memory material and are configured to bend spontaneously for displacing radially away from said tube (6) when they exit from said outer catheter (1), making said annular slider (4) slide around the tube (6) towards said proximal ends, said wire elements (5) being configured to be in abutment against inner walls of the blood vessel at opposite points contrasting displacements of said tube (6);
wherein the outer catheter (1) has a respective first handle (M1) configured to push the outer catheter forward inside a blood vessel and to make said outer catheter (1) slide on the intermediate catheter (2),
**characterized in that**
the intermediate catheter (2) has a respective second handle (M2) configured to push forward and/or pull backward said intermediate catheter (2) in said outer catheter (1);
the inner catheter (3) has a respective third handle (M3) configured to push forward and/or pull backward said inner catheter (3) in said intermediate catheter (2) and to rotate it around a longitudinal axis, said third handle (M3) having a through hole to allow insertion of a guidewire (7) from back, wherein when the first handle (M1) is retracted against the second handle (M2), the anchoring element protrudes from the outer catheter (1);
wherein at least an apical portion of said inner catheter (3) is configured to assume a curved shape at rest, said apical portion being configured to be elastically distended when inserted in the tube (6) of said intermediate catheter (2) and to assume spontaneously the curved shape at rest as far as it protrudes out of the tube (6), so as the apical part of the inner catheter (3) is elastically forced to remain lying when it is inside the intermediate catheter (2) and, when the third handle (M3) is pushed towards the second handle (M2), the apical part of the inner catheter (3) comes out of the tube (6) and the longer the inner catheter (3) protrudes from the tube (6) the more it bends so as, by pushing or retracting the third handle (M3) and by turning it on itself, an operator may adjust at will a curvature of the apical part of the inner catheter (3) and orient in the blood vessel the apical part of the inner catheter (3) in order to point it.

2. The device according to one of claim 1, wherein said annular slider (4) is integral with said wire elements (5) in correspondence of their distal ends.

3. The device according to one of claims 1 or 2, wherein said wire elements (5) are made of a shape memory material suitable for being put in contact with tissues of a human body.

4. The device according to one of claims from 1 to 3, further comprising a wire guide (7) for hearth valve prosthesis inserted in said inner catheter (3).

5. The device according to one of claims from 1 to 4, wherein said wire elements (5) and said apical portion of the inner catheter (3) are made of Nitinol.

6. The device according to one of claims 1 to 5, wherein said inner catheter (3) is made of a shape memory material.

## Patentansprüche

1. Eine Vorrichtung zum Einsetzen eines Führungsdrahtes für eine Herzklappenprothese in ein Blutgefäß, umfassend:
- einen äußeren Katheter (1),
- einen mittleren Katheter (2), der in den besagten äußeren Katheter (1) eingeführt werden kann,
- einen inneren Katheter (3), der in den besagten mittleren Katheter (2) eingeführt werden kann,
- wobei der besagte mittlere Katheter (2) einen apikalen Bereich aufweist, der einen Schlauch (6) begrenzt, auf welchem ein Verankerungselement zur Verankerung an den Innenwänden eines Blutgefäßes gleitet, wobei das besagte Verankerungselement einen ringförmigen Schieber (4) umfasst, welcher den besagten Schlauch (6) verschiebbar umgibt, sowie eine Mehrzahl von Drahtelementen (5), von denen jedes ein an dem besagten Schlauch (6) festgelegtes, proximales Ende aufweist und ein distales Ende, das an dem besagten, ringförmigen Schieber (4) befestigt ist, wobei die besagten Drahtelemente (5) aus einem Formgedächtniswerkstoff bestehen und dazu ausgelegt, sich spontan zu verbiegen, um sich von dem besagten Schlauch (6) radial weg zu verlagern, wenn sie aus dem besagten äußeren Katheter (1) austreten, wobei sie den besagten ringförmigen Schieber (4) zum Gleiten entlang des Schlauchs (6) in Richtung zu den besagten proximalen Enden veranlassen, wobei die besagten Drahtelemente (5) derart gestaltet sind, dass sie an einander gegenüber liegenden Punkten an den inneren Wänden des Blutgefäßes anliegen, um Verlagerungen des besagten Schlauchs (6) zu kontrastieren;
- wobei der äußere Katheter (1) einen entsprechenden ersten Griff (M1) aufweist, der dazu ausgelegt ist, den äußeren Katheter in ein Blutgefäß hinein zu schieben und den besagten äußeren Katheter (1) auf dem mittleren Katheter (2) ins Gleiten zu bringen;
**dadurch gekennzeichnet, dass**
- der mittlere Katheter (2) einen entsprechenden zweiten Griff (M2) aufweist, der dazu ausgelegt ist, den mittleren Katheter (2) innerhalb des besagten äußeren Katheters (1) nach vorne zu schieben und/oder zurück zu ziehen;
- der innere Katheter (3) einen entsprechenden dritten Griff (M3) aufweist, der dazu ausgelegt ist, den inneren Katheter (3) im mittleren Katheter (2) nach vorne zu schieben und/oder zurück zu ziehen, und ihn um eine Längsachse rotieren zu lassen, wobei der besagte dritte Griff (M3) eine durchgehende Öffnung aufweist, um das Einsetzen eines Führungsdrahtes (7) von hinten zu erlauben, wobei das Verankerungselement aus dem äußeren Katheter (1) hervorsteht, wenn der erste Griff (M1) gegenüber dem zweiten Griff (M2) zurückgezogen wird;
- wobei wenigstens ein apikaler Bereich des besagten inneren Katheters (3) dazu ausgelegt ist, im Ruhezustand eine gebogene Form anzunehmen, wobei der besagte apikale Bereich dazu ausgelegt ist, sich elastisch auszudehnen, wenn er in den Schlauch (6) des besagten mittleren Katheters (2) eingeführt wird, und im Ruhezustand spontan die gebogene Form anzunehmen, sofern er aus dem Schlauch (6) herausragt, so dass der apikale Bereich des inneren Katheters (3) elastisch in eine Liegeposition gezwungen wird, und wenn er sich im Inneren des mittleren Katheters (2) befindet, und wenn der dritte Griff (M3) in Richtung des zweiten Griffes (M2) verschoben wird, kommt der apikale Teil des inneren Katheters (3) aus dem Schlauch (6) heraus, und je länger der innere Katheter (3) aus dem Schlauch (6) herausragt, desto mehr verbiegt er sich, so dass durch das Schieben oder Zurückziehen des dritten Griffes (M3) und durch dessen Verdrehen ein Anwender eine Biegung des apikalen Bereichs des inneren Katheters (3) willkürlich einstellen und innerhalb des Blutgefäßes den apikalen Bereich des inneren Katheters (3) orientieren kann, um ihn auszurichten.

2. Die Vorrichtung gemäß Anspruch 1, wobei der besagte ringförmige Schieber (4) mit den besagten Drahtelementen (5) integral ausgeführt ist, in Verbindung mit deren distalen Enden.

3. Die Vorrichtung gemäß einem der Ansprüche 1 oder 2, wobei die besagten Drahtelemente (5) aus einem Formgedächtniswerkstoff bestehen, der dazu geeignet ist, in Kontakt mit Gewebe eines menschlichen Körpers gebracht zu werden.

4. Die Vorrichtung gemäß einem der Ansprüche von 1 bis 3, ferner umfassend eine in dem besagten inneren Katheter (3) eingesetzte Drahtführung (7) für eine Herzklappenprothese.

5. Die Vorrichtung gemäß einem der Ansprüche von 1 bis 4, wobei die besagten Drahtelemente (5) und der besagte apikale Bereich des inneren Katheters (3) aus Nitinol bestehen.

6. Die Vorrichtung gemäß einem der Ansprüche 1 bis 5, wobei der besagte Innenkatheter (3) aus einem Formgedächtniswerkstoff besteht.

## Revendications

1. Dispositif pour insérer un fil de guidage approprié pour une prothèse de valvule cardiaque dans un vaisseau sanguin, comprenant :
un cathéter externe (1),
un cathéter intermédiaire (2) qui peut être inséré dans ledit cathéter externe (1),
un cathéter interne (3) qui peut être inséré dans ledit cathéter intermédiaire (2),
dans lequel ledit cathéter intermédiaire (2) a une partie apicale qui définit un tube (6) sur lequel un élément d'ancrage pour s'ancrer aux parois internes d'un vaisseau sanguin, coulisse, ledit élément d'ancrage comprenant une glissière annulaire (4) pouvant coulisser autour dudit tube (6) et une pluralité d'éléments de fil (5) ayant chacun une extrémité proximale fixée audit tube (6) et une extrémité distale fixée à ladite glissière annulaire (4), dans lequel lesdits éléments de fil (5) sont réalisés avec un matériau à mémoire de forme et sont configurés pour se plier spontanément afin de se déplacer radialement à l'opposé dudit tube (6) lorsqu'ils sortent dudit cathéter externe (1), permettant à ladite glissière annulaire (4) de coulisser autour du tube (6) vers lesdites extrémités proximales, lesdits éléments de fil (5) étant configurés pour être en butée contre des parois internes du vaisseau sanguin au niveau des points opposés contrastant des déplacements dudit tube (6) ;
dans lequel le cathéter externe (1) a une première poignée (M1) respective configurée pour pousser le cathéter externe vers l'avant à l'intérieur d'un vaisseau sanguin et permettre audit cathéter externe (1) de coulisser sur le cathéter intermédiaire (2),
**caractérisé en ce que** :
le cathéter intermédiaire (2) a une deuxième poignée (M2) respective configurée pour pousser vers l'avant et/ou tirer vers l'arrière ledit cathéter intermédiaire (2) dans ledit cathéter externe (1) ;
le cathéter interne (3) a une troisième poignée (M3) respective configurée pour pousser vers l'avant et/ou tirer vers l'arrière ledit cathéter interne (3) dans ledit cathéter intermédiaire (2) et le faire tourner autour d'un axe longitudinal, ladite troisième poignée (M3) ayant un trou débouchant pour permettre l'insertion d'un fil de guidage (7) depuis l'arrière, dans lequel lorsque la première poignée (M1) est rétractée contre la deuxième poignée (M2), l'élément d'ancrage fait saillie du cathéter externe (1) ;
dans lequel au moins une partie apicale dudit cathéter interne (3) est configurée pour adopter une forme incurvée au repos, ladite partie apicale étant configurée pour être élastiquement détendue lorsqu'elle est insérée dans le tube (6) dudit cathéter intermédiaire (2) et pour adopter spontanément la forme incurvée au repos tant qu'elle fait saillie hors du tube (6), de sorte que la partie apicale du cathéter interne (3) est élastiquement forcée à rester couchée lorsqu'elle est à l'intérieur du cathéter intermédiaire (2) et lorsque la troisième poignée (M3) est poussée vers la deuxième poignée (M2), la partie apicale du cathéter interne (3) sort du tube (6) et plus le cathéter interne (3) fait saillie du tube (6), plus il se plie de sorte que, en poussant ou en rétractant la troisième poignée (M3) ou en la faisant tourner sur elle-même, un opérateur peut ajuster, à volonté, une courbure de la partie apicale du cathéter interne (3) et orienter dans le vaisseau sanguin, la partie apicale du cathéter interne (3) afin de la diriger.

2. Dispositif selon la revendication 1, dans lequel ladite glissière annulaire (4) est solidaire avec lesdits éléments de fil (5) en correspondance de leurs extrémités distales.

3. Dispositif selon l'une des revendications 1 ou 2, dans lequel lesdits éléments de fil (5) sont réalisés à partir d'un matériau à mémoire de forme approprié pour être mis en contact avec les tissus d'un corps humain.

4. Dispositif selon l'une des revendications 1 à 3, comprenant en outre un guide de fil (7) pour une prothèse de valvule cardiaque insérée dans ledit cathéter interne (3).

5. Dispositif selon l'une des revendications 1 à 4, dans lequel lesdits éléments de fil (5) et ladite partie apicale du cathéter interne (3) sont réalisés à partir de Nitinol.

6. Dispositif selon l'une des revendications 1 à 5, dans lequel ledit cathéter interne (3) est réalisé avec un matériau à mémoire de forme.
